# EUROPEAN PATENT SPECIFICATION

(11) **EP 2 051 730 B1**
(45) Date of publication and mention of the grant of the patent: **02.11.2011**
(21) Application number: 07814209.8
(22) Date of filing: 17.08.2007
(51) Int. Cl.: A61K 38/16, A61P 31/16

(54) **ANTI-H5N1 INFLUENZA ACTIVITY OF THE ANTIVIRAL PROTEIN CYANOVIRIN**
ANTI-H5N1-INFLUENZA-AKTIVITÄT DES ANTIVIRALEN PROTEINS CYANOVIRIN
ACTIVITE ANTIGRIPPE ANTI-H5N1 DE LA PROTEINE ANTIVIRALE CYANOVIRINE

(30) Priority: 18.08.2006 US 838712 P
(43) Date of publication of application: 29.04.2009
(73) Proprietor: Government of the United States of America, Represented by the Secretary, Department of Health and Human Services, Rockville, Maryland 20852 (US)
(72) Inventor: O'KEEFE, Barry R., Frederick, Maryland 21702 (US); MCMAHON, James B., Frederick, Maryland 21701 (US)
(74) Representative: Grünecker, Kinkeldey, Stockmair & Schwanhäusser
(86) International application number: PCT/US2007/076181
(87) International publication number: WO 2008/022303

(56) References cited:
- US-A1- 2002 127 675
- O'KEEFE BARRY R ET AL: "Potent anti-influenza activity of cyanovirin-N and interactions with viral hemagglutinin" ANTIMICROBIAL AGENTS AND CHEMOTHERAPY, AMERICAN SOCIETY FOR MICROBIOLOGY, WASHINGTON, DC, US, vol. 47, no. 8, August 2003 (2003-08), pages 2518-2525, XP002438756 ISSN: 0066-4804 cited in the application
- STEPHENSON IAIN ET AL: "Influenza: current threat from avian influenza" BRITISH MEDICAL BULLETIN, vol. 75-76, 2005, pages 63-80, XP002467263 ISSN: 0007-1420

### SEQUENCE LISTING

<110> GOVERNMENT OF THE UNITED STATES OF AMERICA, REPRESENTED BY
   THE SECRETARY, DEPARTMENT OF HEALTH AND HUMAN SERVICES
<120> ANTI-H5N1 INFLUENZA ACTIVITY OF THE ANTIVIRAL PROTEIN CYANOVIRIN
<130> EP63962FZpau
<140> EP 07 814 209.8 <141> 2007-08-17
<150> PCT/US07/76181 <151> 2007-08-17
<150> US 60/838,712 <151> 2006-08-18
<160> 2
<170> PatentIn version 3.5
<210> 1
   <211> 101
   <212> PRT
   <213> Nostoc ellipsosporum
<220>
   <221> misc_feature
   <222> (30)..(30)
   <223> xaa = Asn, Ala, Glu, val, or no amino acid.
<220>
   <221> misc_feature
   <222> (51)..(51)
   <223> Xaa = Pro, Gly, or no amino acid.
<400> 1
   Leu Gly Lys Phe Ser Gln Thr Cys Tyr Asn Ser Ala Ile Gln Gly Ser
   1               5                   10                  15
   Val Leu Thr Ser Thr Cys Glu Arg Thr Asn Gly Gly Tyr Xaa Thr Ser
               20                  25                  30
   Ser Ile Asp Leu Asn Ser Val Ile Glu Asn Val Asp Gly Ser Leu Lys
           35                  40                  45
   Trp Gln Xaa Ser Asn Phe Ile Glu Thr Cys Arg Asn Thr Gln Leu Ala
       50                  55                  60
   Gly Ser Ser Glu Leu Ala Ala Glu Cys Lys Thr Arg Ala Gln Gln Phe
   65                  70                  75                  80
   Val Ser Thr Lys Ile Asn Leu Asp Asp His Ile Ala Asn Ile Asp Gly
                   85                  90                  95
   Thr Leu Lys Tyr Glu
               100
<210> 2
   <211> 101
   <212> PRT
   <213> Nostoc ellipsosporum
<400> 2
   Leu Gly Lys Phe Ser Gln Thr Cys Tyr Asn Ser Ala Ile Gln Gly Ser
   1               5                   10                  15
   Val Leu Thr Ser Thr Cys Glu Arg Thr Asn Gly Gly Tyr Asn Thr Ser
               20                  25                  30
   Ser Ile Asp Leu Asn Ser Val Ile Glu Asn Val Asp Gly Ser Leu Lys
           35                  40                  45
   Trp Gln Pro Ser Asn Phe Ile Glu Thr Cys Arg Asn Thr Gln Leu Ala
       50                  55                  60
   Gly Ser Ser Glu Leu Ala Ala Glu Cys Lys Thr Arg Ala Gln Gln Phe
   65                  70                  75                  80
   Val Ser Thr Lys Ile Asn Leu Asp Asp His Ile Ala Asn Ile Asp Gly
                   85                  90                  95
   Thr Leu Lys Tyr Glu
               100

## Description

### BACKGROUND OF THE INVENTION

The avian influenza A (H5N1) epizootic (animal outbreak) in Asia and parts of Europe, the Near East, and Africa is not expected to diminish significantly in the short term. It is likely that H5N1 infection among birds has become endemic in certain areas and that human infections resulting from direct contact with infected poultry will continue to occur. Thus far, the spread of H5N1 virus from person-to-person has been rare and has not continued beyond one person. Although avian influenza A viruses usually do not infect humans, more than 200 confirmed cases of human infection with avian influenza viruses have been reported since 1997.

Four different influenza antiviral drugs (amantadine, rimantadine, oseltamivir, and zanamivir) are approved by the U.S. Food and Drug Administration (FDA) for the treatment and prevention of influenza. All four have activity against influenza A viruses (Stephenson et al., British Medical Bulletin, vol.75-76,2005, pagen 63-80). However, influenza strains can become resistant to these drugs, and therefore the drugs are not always effective. For example, analyses of some of the 2004 H5N1 viruses isolated from poultry and humans in Asia have shown that the viruses are resistant to two of the medications (amantadine and rimantadine).

Moreover, H5N1 viruses infect cells primarily found deep within human lungs rather than in the upper respiratory tract, as occurs in most human influenza infections (Sinya et al., Nature, 440(7083): 435-436 (2006); van Riel et al., Science, 312(5772): 399 (2006)). The fact that H5N1 infects cells of the lower respiratory tract restricts the virus's ability to be passed person-to-person via the droplet route (e.g., coughing and sneezing), but also makes treatment of the infection, which often leads to the development of severe pneumonia, more difficult.

Accordingly, it is an object of the invention to provide new methods for inhibiting prophylactically or therapeutically an H5N1 viral infection of a host. This and other objects and advantages of the invention, as well as additional inventive features, will become apparent from the description provided herein.

### BRIEF SUMMARY OF THE INVENTION

The invention provides the use of an anti-viral effective amount of an antiviral protein comprising the amino acid sequence of cyanovirin, an antiviral portion or conjugate thereof for the preparation of a medicament for inhibiting prophylactically or therapeutically an H5N1 viral infection in a host,

The invention also provides the use of an anti-viral effective amount of a nucleic acid molecule that encodes the antiviral protein comprising the amino acid sequence of cyanovirin, an antiviral portion or conjugate thereof, such as in a vector or host cell for the preparation of a medicament for inhibiting prophylactically or therapeutically an H5N1 viral infection in a host,

### DETAILED DESCRIPTION OF THE INVENTION

Cyanovirin and related conjugates, compositions, nucleic acids, vectors, and host cells have been described previously (see, e.g., Boyd et al., Antimicrobial Agents and Chemotherapy, 41(7): 1521-1530 (1997); and U.S. Patent No. 6,780,847). Similarly, the use of cyanovirin for treatment of certain strains of influenza virus previously has been described (see, e.g., O'Keefe et al., Antimicrobial Agents and Chemotherapy, 47(8): 2518-2825 (2003); and U.S. Patent No. 6,780,847). However, it was not known whether cyanovirin would be effective for the treatment of avian influenza viruses that infect humans, such as avian influenza A (H5N1).

The inventors discovered that cyanovirin has activity against H5N1 avian influenza strains, including the strains that infect humans (i.e., humanized H5N1). uses for Accordingly, the invention provides uses for inhibiting prophylactically or therapeutically an H5N1 viral infection in a host. The uses include administering to the host an anti-viral effective amount of an antiviral protein comprising the amino acid sequence of cyanovirin, an antiviral portion uses, or conjugate thereof. Additionally, the uses include administering to the host an anti-viral effective amount of a nucleic acid molecule that encodes the antiviral protein comprising the amino acid sequence of cyanovirin, an antiviral portion, or conjugate thereof, such as in a vector or host cell.

Herein the term "cyanovirin" is used generically to refer to a native cyanovirin or any related, functionally equivalent (i.e., antiviral) protein, or a variant or portion (i.e., fragment) of cyanovirin, as well as nucleic acid molecules that encode a native cyanovirin or any related, functionally equivalent (i.e., antiviral) protein, a variant or portion of cyanovirin, and conjugates thereof. A related, functionally equivalent protein, or a variant or portion of cyanovirin, (a) contains a sequence of at least nine amino acids identical to nine contiguous amino acids contained within a native cyanovirin, and (b) is capable of specifically binding to a virus, in particular an influenza virus (e.g., H5N1) or a retrovirus (e.g., a primate immunodeficiency virus, such as HIV-1, HIV-2 or SIV), or to an infected host cell expressing one or more viral antigen(s) (e.g., an envelope glycoprotein) of the respective virus. A functionally equivalent protein can comprise the amino acid sequence of a native cyanovirin, particularly cyanovirin-N (see SEQ ID NOs: 1 and 2), in which 1-20, preferably 1-10, more preferably 1, 2, 3, 4, or 5, and most preferably 1 or 2, amino acids have been removed from one or both ends, preferably from only one end, and most preferably from the amino-terminal end, of the native cyanovirin. Alternatively, a functionally equivalent protein can comprise the amino acid sequence of a native cyanovirin, particularly cyanovirin-N (see SEQ ID NOs: 1 and 2), in which 1-20, preferably 1-10, more preferably 1, 2, 3, 4, or 5, and most preferably 1 or 2, amino acids have been added to one or both ends, preferably from only one end, and most preferably from the amino-terminal end, of the native cyanovirin.

Preferably, the protein, variant, or portion thereof comprises an amino acid sequence that is substantially identical to that of an antiviral protein from *Nostoc ellipsosporum.* By "substantially identical" is meant sufficient identity to render the protein, or variant or portion thereof, antiviral, with antiviral activity characteristic of an antiviral protein isolated *from Nostoc ellipsosporum.* The protein should exhibit at least about 50% (e.g., at least about 55%, at least about 60%, at least about 65%, or at least about 70%) identity, preferably at least about 75% (e.g., at least about 80% or at least about 90%) identity, and most preferably at least about 90% (e.g., at least about 95%, at least about 96%, at least about 97%, at least about 98%, or at least about 99%) identity.

A cyanovirin conjugate comprises a cyanovirin coupled to one or more selected effector molecule(s), such as a toxin or immunological reagent. "Immunological reagent" refers to an antibody, an immunoglobulin, and an immunological recognition element. An immunological recognition element is an element, such as a peptide, e.g., the FLAG sequence of the recombinant cyanovirin-FLAG fusion protein, which facilitates, through immunological recognition, isolation, purification, and/or analysis of the protein to which it is attached. A cyanovirin fusion protein is a type of cyanovirin conjugate, wherein a cyanovirin is coupled to one or more other protein(s) having any desired properties or effector functions, such as cytotoxic or immunological properties, or other desired properties, such as to facilitate isolation, purification, or analysis of the fusion protein.

Specific cyanovirins to be administered in the inventive uses include cyanovirins comprising the amino acid sequence of SEQ ID NO: 1, as well as an antiviral portion, variant, or conjugates thereof. For example, the cyanovirin can comprise the amino acid sequence of SEQ ID NO: 2, which represents cyanovirin-N. Preferably, amino acids 30-32 of SEQ ID NO: 1 have been rendered glycosylation resistant. Amino acids 30-32 of SEQ ID NO: 1 can be rendered glycosylation resistant by deletion of amino acid residue 30 or use of an amino acid other than asparagine for amino acid residue 30. Preferably, amino acid residue 30 is an amino acid selected from the group consisting of alanine, glutamine, and valine.

Additionally or alternatively, the proline at amino acid 51 of SEQ ID NO: 1 can be deleted or be an amino acid other than proline (e.g., glycine). Substitution or deletion of the proline at position 51 of SEQ ID NO: 2 is believed to enhance the dimerization resistance. Dimerization of a cyanovirin is known to occur under certain conditions (Yang et al., J. Molec. Biol., 288: 403-412 (1999); and Bewley et al., JACS, 12: 6009-6016 (2000)), and folding stability and resistance to physiochemical degradation are desirable additional attributes of the cyanovirins. Such traits are desirable for large-scale isolation and purification and mass production of cyanovirins in prokaryotic and eukaryotic host-cells/organisms.

Accordingly, the inventive uses comprise administering cyanovirins comprising the amino acid sequence of SEQ ID NO: 1, or an antiviral portion wherein amino acid 30 is selected from the group consisting of alanine, guanine, and valine or wherein amino acid 30 is deleted. Additionally, the inventive uses comprise administering cyanovirins comprising the amino acid sequence of SEQ ID NO: 1, or an antiviral portion, wherein amino acid 51 of SEQ ID NO: 1 is glycine or is deleted.

The antiviral portions of cyanovirin to be administered in the inventive uses preferably comprise at least nine contiguous amino acids of the amino acid sequence of SEQ ID NO: 1, which desirably have antiviral activity. If the at least nine contiguous amino acids comprise amino acids 30-32 of SEQ ID NO: 1, desirably amino acids 30-32 have been rendered glycosylation resistant (e.g., by selecting amino acid 30 from the group consisting of alanine, guanine, and valine, or by deleting amino acid 30 as described above), yet maintain antiviral activity. If the at least nine contiguous amino acids of SEQ ID NO: 1 comprise amino acid 51 of SEQ ID NO: 1, desirably amino acid 51 is glycine or is deleted, as described above.

Given the present disclosure, it will be apparent to one skilled in the art that a partial cyanovirin-N gene sequence will likely suffice to code for a fully functional, i.e., antiviral, such as anti-H5N1, cyanovirin. A minimum essential DNA coding sequence(s) for a functional cyanovirin can readily be determined by one skilled in the art, for example, by synthesis and evaluation of sub-sequences comprising the native cyanovirin, and by site-directed mutagenesis studies of the cyanovirin-N DNA coding sequence.

The anti-viral, e.g., anti-H5N1, activity of the cyanovirins can be demonstrated by assays, such as the neutral red, visual, and virus yield assays set forth in the Example, which accurately predict for antiviral activity in animals, such as humans. These assays measure the ability of compounds to prevent the replication of virus and/or the cytopathic effects of the virus on human target cells. These measurements directly correlate with the pathogenesis of H5N1-induced disease *in vivo.*

The proteins and nucleic acids for use in the preparation of a medicament according to the invention can be obtained by any suitable manner, including isolation and purification of the native protein or nucleic acid from *Nostoc ellipsosporum* or by synthetic or recombinant means.

When the cyanovirin is administered as a nucleic acid, the inventive uses comprise administering a vector comprising a DNA sequence, e.g., a *Nostoc ellipsosporum* gene sequence for cyanovirin, a cDNA encoding a cyanovirin, or a synthetic DNA sequence encoding cyanovirin, and a host cell comprising the vector. The vector comprising the DNA sequence can be any suitable vector, such as plasmids, plasmid-liposome complexes, and viral vectors, e.g., parvoviral-based vectors (i.e., adeno-associated virus (AAV)-based vectors), retroviral vectors, herpes simplex virus (HSV)-based vectors, AAV-adenoviral chimeric vectors, and adenovirus-based vectors. Any of these vectors can be prepared using standard recombinant DNA techniques described in, e.g., Ausubel et al., Current Protocols in Molecular Biology, Greene Publishing Associates and John Wiley & Sons, New York, N.Y. (1994).

The DNA, whether isolated and purified or synthetic, or cDNA encoding a cyanovirin can encode either the entire cyanovirin or a portion thereof. Where the DNA or cDNA does not comprise the entire coding sequence of the native cyanovirin, the DNA or cDNA can be subcloned as part of a gene fusion. In a transcriptional gene fusion, the DNA or cDNA contains its own control sequence directing appropriate production of protein (e.g., ribosome binding site, translation initiation codon, etc.), and the transcriptional control sequences (e.g., promoter elements and/or enhancers) can be provided by the vector. In a translational gene fusion, transcriptional control sequences as well as at least some of the translational control sequences (i.e., the translational initiation codon) can be provided by the vector. In the case of a translational gene fusion, a chimeric protein can be produced.

Nucleic acids can be constructed for specific fusion proteins containing a functional cyanovirin component plus a fusion component conferring additional desired attribute(s) to the composite protein. For example, a fusion sequence for a toxin or immunological reagent, as defined above, can be added to facilitate purification and analysis of the functional protein (e.g., such as the FLAG-cyanovirin-N fusion protein). The nucleic acids also can be constructed to code for fusion proteins, which contain a cyanovirin coupled to an effector protein, such as a toxin or immunological reagent, for specific targeting to a virus or viral-infected cells, e.g., H5N1 influenza virus and/or H5N1-infected cells. In these instances, the cyanovirin moiety serves not only as a neutralizing agent but also as a targeting agent to direct the effector activities of these molecules selectively against a given virus, such as H5N1 influenza virus. Thus, for example, a therapeutic agent can be obtained by combining the H5N1-targeting function of a functional cyanovirin with a toxin aimed at neutralizing infectious virus and/or by destroying cells producing infectious virus, such as H5N1 influenza virus. Similarly, a therapeutic agent can be obtained, which combines the viral-targeting function of a cyanovirin with the multivalency and effector functions of various immunoglobulin subclasses.

Viral-targeted conjugates can be prepared either by genetic engineering techniques (see, for example, Chaudhary et al., Nature, 335: 369-372 (1988)) or by chemical coupling of the targeting component with an effector component. The most feasible or appropriate technique to be used to construct a given cyanovirin conjugate or fusion protein will be selected based upon consideration of the characteristics of the particular effector molecule selected for coupling to a cyanovirin. For example, with a selected non-proteinaceous effector molecule, chemical coupling, rather than genetic engineering techniques, can be the only feasible option for creating the desired cyanovirin conjugate.

The coupling can be effected at the DNA level or by chemical coupling. For example, a cyanovirin-effector protein conjugate can be obtained by (a) selecting a desired effector protein or peptide; (b) synthesizing a composite DNA coding sequence comprising a first DNA coding sequence comprising one of the aforementioned nucleic acid sequences, which codes for a functional cyanovirin, coupled to a second DNA coding sequence for an effector protein or peptide, e.g., a toxin or immunological reagent; (c) expressing the composite DNA coding sequence in an appropriate protein-synthesizing organism; and (d) purifying the desired fusion protein or peptide to substantially pure form. Alternatively, a cyanovirin-effector molecule conjugate can be obtained by (a) selecting a desired effector molecule and a cyanovirin or cyanovirin fusion protein; (b) chemically coupling the cyanovirin or cyanovirin fusion protein to the effector molecule; and (c) purifying the desired cyanovirin-effector molecule conjugate to substantially pure form.

Other functional reagents that can be used as effector components in the conjugates can include, for example, polyethylene glycol, dextran, albumin, and the like, whose intended effector functions may include one or more of the following: to improve stability of the conjugate; to increase the half-life of the conjugate; to increase resistance of the conjugate to proteolysis; to decrease the immunogenicity of the conjugate; to provide a means to attach or immobilize a functional cyanovirin onto a solid support matrix (e.g., see, for example, Harris, in Poly(Ethylene Glycol) Chemistry: Biotechnical and Biomedical Applications, Harris, ed., Plenum Press: New York (1992), pp. 1-14). Conjugates furthermore can comprise a functional cyanovirin coupled to more than one effector molecule, each of which, optionally, can have different effector functions (e.g., such as a toxin molecule (or an immunological reagent) and a polyethylene glycol (or dextran or albumin) molecule). Diverse applications and uses of functional proteins and peptides, such as in the present instance a functional cyanovirin, attached to or immobilized on a solid support matrix, are exemplified more specifically for poly(ethylene glycol) conjugated proteins or peptides in a review by Holmberg et al. (in Poly(Ethylene Glycol) Chemistry Biotechnical and Biomedical Applications, Harris, ed., Plenum Press: New York, 1992, pp. 303-324). Preferred examples of solid support matrices include magnetic beads and a flow-through matrix.

The appropriate delivery system for a given cyanovirin depends upon its particular nature, the particular clinical application, and the site of drug action. As with any protein or peptide drug, oral delivery of a cyanovirin can present special problems, due primarily to its instability in the gastrointestinal tract and the poor absorption and bioavailability of intact, bioactive drug therefrom. Therefore, especially in the case of oral delivery, but also possibly in conjunction with other routes of delivery, it could be necessary to use an absorption-enhancing agent in combination with a given cyanovirin. A wide variety of absorption-enhancing agents have been investigated and/or applied in combination with protein and peptide drugs for oral delivery and for delivery by other routes. Most commonly, typical enhancers fall into the general categories of (a) chelators, such as EDTA, salicylates, and N-acyl derivatives of collagen, (b) surfactants, such as lauryl sulfate and polyoxyethylene-9-lauryl ether, (c) bile salts, such as glycholate and taurocholate, and derivatives, such as taurodihydrofusidate, (d) fatty acids, such as oleic acid and capric acid, and their derivatives, such as acylcarnitines, monoglycerides, and diglycerides, (e) non-surfactants, such as unsaturated cyclic ureas, (f) saponins, (g) cyclodextrins, and (h) phospholipids.

Other approaches to enhancing the oral delivery of protein and peptide drugs, such as the cyanoviriris, can include chemical modifications to enhance stability to gastrointestinal enzymes and/or increased lipophilicity. Alternatively, or in addition, the protein or peptide drug can be administered in combination with other drugs or substances, which directly inhibit proteases and/or other potential sources of enzymatic degradation of proteins and peptides. Yet another alternative approach to prevent or delay gastrointestinal absorption of protein or peptide drugs, such as cyanovirins, is to incorporate them into a delivery system that is designed to protect the protein or peptide from contact with the proteolytic enzymes in the intestinal lumen and to release the intact protein or peptide only upon reaching an area favorable for its absorption. A more specific example of this strategy is the use of biodegradable microcapsules or microspheres, both to protect vulnerable drugs from degradation, as well as to effect a prolonged release of active drug (Deasy, in Microencapsulation and Related Processes, Swarbrick, ed., Marcell Dekker, Inc.: New York, 1984, pp. 1-60, 88-89, 208-211). Microcapsules also can provide a useful way to effect a prolonged delivery of a protein and peptide drug, such as a cyanovirin or conjugate thereof, after injection (Maulding, J. Controlled Release, 6: 167-176 (1987)).

There are numerous other potential routes of delivery of the cyanovirin. These routes include intravenous, intraarterial, intrathecal, intracistemal, buccal, rectal, nasal, pulmonary, transdermal, vaginal, ocular, and the like. To protect against a possible immunogenic reaction, the cyanovirin can be modified to mask immunogenic groups. It also is possible to protect against undesired immune responses by judicious choice of the method of formulation and/or administration. For example, site-specific delivery can be employed, as well as masking of recognition sites from the immune system by use or attachment of a so-called tolerogen, such as polyethylene glycol, dextran, albumin, and the like. Such modifications also can have advantageous effects on stability and half-life both *in vivo* and *ex vivo*.

Procedures for covalent attachment of molecules, such as polyethylene glycol, dextran, albumin, and the like, to proteins, such as cyanovirins, are well-known to those skilled in the art, and are extensively documented in the literature (see, e.g., Davis et al., in Peptide and Protein Drug Delivery, Lee, ed., Marcel Dekker: New York, 1991, pp. 831-864).

Other strategies to avoid untoward immune reactions can also include the induction of tolerance by administration initially of only low doses. In any event, it will be apparent from the present disclosure to one skilled in the art that, for any particular desired medical application or use of a cyanovirin, the skilled artisan can select what is advantageous from any of a wide variety of possible compositions, routes of administration, or sites of application.

The cyanovirin can be administered alone or in a composition (e.g., a pharmaceutical composition). The composition can comprise a carrier, such as a pharmaceutically acceptable carrier. Pharmaceutically acceptable carriers are well-known to those who are skilled in the art. The choice of carrier will be determined in part by the particular cyanovirin or conjugate thereof, as well as by the particular method used to administer the composition.

The composition further can comprise at least one additional antiviral (e.g., antiinfluenza) compound other than a cyanovirin, such as in an anti-viral effective amount. Suitable antiviral compounds include M2 ion channel inhibitors (e.g., amantadine, rimantadine, and or a combination thereof) and neuramidase inhibitors (e.g., oseltamivir, zanamivir, or a combination thereof), entry inhibitors (e.g., Griffithsin, which is described in, e.g., Mori et al., J. Biol. Clam., 280(10): 9345-9353 (2005)), Enfurtide (i.e., Enfuvirtide^{™} or T-20), and combinations thereof. The composition also can comprise an immunoadjuvant, such as polyphosphazene polyelectrolyte. Cyanovirin also can be combined with other compounds, such as virucides, immunomodulators, immunostimulants, antibiotics, antifungals, and absorption enhancers.

The cyanovirin can be administered to any suitable host to prophylactically or therapeutically inhibit a H5N1 viral infection (e.g., by inhibiting the growth or replication of H5N1 influenza virus). Suitable hosts include animals, such as birds (e.g., ducks, gulls, chickens) and mammals, such as ferrets, cats (e.g., domestic cats, tigers, leopards), dogs, pigs, horses, seals, whales, and humans, who are infected with the virus or who are at risk for viral infection. Cyanovirin also can be used to treat objects or materials, such as medical equipment, supplies, or fluids, including biological fluids, such as blood, blood products and vaccine formulations, cells, tissues, and organs, to remove or inactivate the virus in an effort to prevent or treat viral infection of an animal, such as a human.

By "therapeutically" is meant that the host already has been infected with the virus. By "prophylactically" is meant that the host has not yet been infected with the virus but is at risk of being infected with the virus. Delivery of the cyanovirin to a location of initial viral contact, such as the nose or mouth, can prophylactically block the onset of viral infection. Prophylactic treatment is intended to encompass any degree of inhibition of viral infection, including, but not limited to, complete inhibition, as one of ordinary skill in the art will readily appreciate that any degree in inhibition of viral infection is advantageous. Preferably, cyanovirin is administered before viral infection or immediately upon determination of viral infection and is continuously administered until the virus is undetectable. The use optionally further comprises the prior, simultaneous, or subsequent administration, by the same route or a different route, of an antiviral compound other than cyanovirin or another active agent that is efficacious in inhibiting the viral infection or opportunistic infections. Upon administration of the anti-viral effective amount of the cyanovirin, the viral infection is inhibited. Cyanovirin inhibits H5N1 viral infection by inhibiting viral binding, fusion, and entry into the host cells and limiting the spread of viral infection by inhibiting cell to cell fusion. Any amount of inhibition, prophylactically or thereapeutically, is desirable and is within the context of the inventive use.

One skilled in the art will appreciate that various routes of administering a drug are available, and, although more than one route can be used to administer a particular drug, a particular route can provide a more immediate and more effective reaction than another route. Furthermore, one skilled in the art will appreciate that the particular pharmaceutical carrier employed will depend, in part, upon the particular cyanovirin employed, and the chosen route of administration.

Formulations suitable for oral administration can consist of liquid solutions, such as an anti-viral effective amount of the compound dissolved in diluents, such as water, saline, or fruit juice; capsules, sachets, or tablets, each containing a predetermined amount of the active ingredient, as solid or granules; solutions or suspensions in an aqueous liquid; and oil-in-water emulsions or water-in-oil emulsions. Tablet forms can include one or more of lactose, mannitol, com starch, potato starch, microcrystalline cellulose, acacia, gelatin, colloidal silicon dioxide, croscarmellose sodium, talc, magnesium stearate, stearic acid, and other excipients, colorants, diluents, buffering agents, moistening agents, preservatives, flavoring agents, and pharmacologically compatible carriers. Suitable formulations for oral delivery also can be incorporated into synthetic and natural polymeric microspheres, or other means can be utilized to protect the active agent(s) from degradation within the gastrointestinal tract (see, for example, Wallace et al., Science, 260: 912-915, (1993)).

Administration of cyanovirins to mucosal layers (e.g., by intranasal, rectal, oral, or vaginal administration) is particularly preferred.

The cyanovirin, alone or in combination with other antiviral compounds, can be made into aerosol formulations or microparticulate powder formulations to be administered via inhalation, such as through the use of an inhaler. These aerosol formulations can be placed into pressurized acceptable propellants, such as dichlorodifluoromethane, propane, nitrogen, and the like.

The cyanovirin, alone or in combinations with other antiviral compounds or absorption modulators, can be made into suitable formulations for transdermal application and absorption. Transdermal electroporation or iontophoresis also can be used to promote and/or control the systemic delivery of the compounds and/or compositions through the skin (e.g., see Theiss et al., Meth. Find. Exp. Clin. Pharmacol., 13: 353-359 (1991)).

Formulations suitable for topical administration include lozenges comprising the active ingredient in a flavor, usually sucrose and acacia or tragacanth; pastilles comprising the active ingredient in an inert base, such as gelatin and glycerin, or sucrose and acacia; and mouthwashes comprising the active ingredient in a suitable liquid carrier; as well as creams, emulsions, gels, and the like containing the active ingredient and a carrier. Suitable carriers are known in the art.

Formulations for rectal administration can be presented as a suppository with a suitable base comprising, for example, cocoa butter, or a salicylate. Formulations suitable for vaginal administration can be presented as pessaries, tampons, creams, gels, pastes, foams, or spray formulas containing the active ingredient and a carrier. Suitable carriers are known in the art.

Formulations suitable for parenteral administration include aqueous and non-aqueous, isotonic sterile injection solutions, which can contain antioxidants, buffers, bacteriostats, and solutes that render the formulation isotonic with the blood of the intended recipient, and aqueous and non-aqueous sterile suspensions that can include suspending agents, solubilizers, thickening agents, stabilizers, and preservatives. The formulations can be presented in unit-dose or multi-dose sealed containers, such as ampules and vials, and can be stored in a freeze-dried (lyophilized) condition requiring only the addition of the sterile liquid carrier, for example, water, for injections, immediately prior to use. Extemporaneous injection solutions and suspensions can be prepared from sterile powders, granules, and tablets of the kind previously described.

Formulations comprising a cyanovirin suitable for virucidal (e.g., H5N1) sterilization of inanimate objects, such as medical supplies or equipment, laboratory equipment and supplies, instruments, devices, and the like, can, for example, be selected or adapted as appropriate, by one skilled in the art, from any of the aforementioned compositions or formulations. Similarly, formulations suitable for *ex vivo* sterilization or removal of virus, such as infectious virus, from a sample, such as blood, blood products, sperm, or other bodily products, such as a fluid, cells, a tissue, or an organ, or any other solution, suspension, emulsion, vaccine formulation (such as in the removal of infectious virus), or any other material which can be administered to a patient in a medical procedure, can be selected or adapted as appropriate by one skilled in the art, from any of the aforementioned compositions or formulations. However, suitable formulations for *ex vivo* sterilization or removal of virus from a sample or on an inanimate object are by no means limited to any of the aforementioned formulations or compositions. For example, a formulation can comprise a functional cyanovirin attached to (e.g., coupled to or immobilized on) a solid support matrix comprising magnetic beads, to facilitate contacting, binding, and removal of infectious virus, and to enable magnet-assisted removal of the virus from a sample, such as a bodily product like a fluid, cells, a tissue, or an organ, e.g., blood, a component of blood, or sperm.

As an even more specific illustration, such a composition (e.g., for *ex vivo* use) can comprise a functional cyanovirin attached to a solid support matrix, such as magnetic beads or a flow-through matrix, by means of an anti-cyanovirin antibody or at least one effector component, which can be the same or different, such as polyethylene glycol, albumin, or dextran. A flow-through matrix can comprise, for instance, a configuration similar to an affinity column. The cyanovirin can be covalently coupled to a solid support matrix via an anti-cyanovirin antibody, as, for example, described below. Methods of attaching an antibody to a solid support matrix are well-known in the art (see, for example, Harlow and Lane. Antibodies: A Laboratory Manual, Cold Springs Harbor Laboratory: Cold Spring Harbor, NY, 1988). Alternatively, the solid support matrix, such as magnetic beads, can be coated with streptavidin, in which case the cyanovirin is biotinylated. Such a composition can be prepared, for example, by biotinylating the cyanovirin and then contacting the biotinylated protein or peptide with a (commercially available) solid support matrix, such as magnetic beads, coated with streptavidin. The use of biotinylation as a means to attach a desired biologically active protein or peptide to a streptavidin-coated support matrix, such as magnetic beads, is well-known in the art.

One skilled in the art will appreciate that.a suitable or appropriate formulation can be selected, adapted, or developed based upon the particular application to be utilized.

For *ex vivo* uses, such as virucidal treatments of inanimate objects or materials, blood or blood products, or tissues, the amount of cyanovirin, or conjugate or composition thereof, to be employed should be sufficient that any virus or virus-producing cells present will be rendered noninfectious or will be destroyed. For example, this would require that the virus and/or the virus-producing cells be exposed to concentrations of cyanovirin-N in the range of about 0.1-1000 nM (e.g., about 0.5 nM, about 1 nM, about 10 nM, about 50 nM, about 100 nM, about 200 nM, about 300 nM, about 400 nM, about 500 nM, about 600 nM, about 700 nM, about 800 nM, about 900 nM, or ranges thereof). Similar considerations apply to *in vivo* applications. Therefore, the designation of "anti-viral effective amount" is used generally to describe the amount of a particular cyanovirin required for antiviral efficacy in any given application.

For *in vivo* uses, the dose of a cyanovirin administered to a host, particularly a human, in the context of the invention should be sufficient to effect a prophylactic or therapeutic response in the individual over a reasonable time frame. The dose used to achieve a desired antiviral concentration *in vivo* (e.g., about 0.1-1000 nM) will be determined by the potency of the particular cyanovirin employed, the severity of the disease state of infected individuals, as well as, in the case of systemic administration, the body weight and age of the infected individual. The size of the dose also will be determined by the existence of any adverse side effects that may accompany the particular cyanovirin employed. It is always desirable, whenever possible, to keep adverse side effects to a minimum.

In terms of the administration of a cyanovirin, the dosage can be in unit dosage form, such as a tablet or capsule. The term "unit dosage form" as used herein refers to physically discrete units suitable as unitary dosages for human and animal subjects, each unit containing a predetermined quantity of a cyanovirin, alone or in combination with other antiviral agents, calculated in an amount sufficient to produce the desired effect in association with a pharmaceutically acceptable diluent, carrier, or vehicle.

The specifications for the unit dosage forms of the invention depend on the particular cyanovirin, or conjugate or composition thereof, employed and the effect to be achieved, as well as the pharmacodynamics associated with each cyanovirin, or conjugate or composition thereof, in the host. The dose administered should be an "anti-viral effective amount" or an amount necessary to achieve an "effective level" in the individual patient.

Since the "effective level" is used as the preferred endpoint for dosing, the actual dose and schedule can vary, depending upon interindividual differences in pharmacokinetics, drug distribution, and metabolism. The "effective level" can be defined, for example, as the blood or tissue level (e.g., about 0.1-1000 nM) desired in the patient that corresponds to a concentration of one or more cyanovirins, which inhibits a virus, such as H5N1, in an assay known to predict for clinical antiviral activity of chemical compounds and biological agents. The "effective level" for the cyanovirin also can vary when the cyanovirin is used in combination with other known antiviral compounds or combinations thereof.

In the treatment of some virally infected individuals, it can be desirable to utilize a "mega-dosing" regimen, wherein a large dose of the cyanovirin is administered, time is allowed for the drug to act, and then a suitable reagent is administered to the individual to inactivate the drug.

It will also be appreciated by one skilled in the art that a DNA sequence of a cyanovirin can be inserted ex *vivo* into mammalian cells previously removed from a given animal, in particular a human, host. Such cells can be employed to express the corresponding cyanovirin *in vivo* after reintroduction into the host. It is also possible that, as an alternative to *ex vivo* insertion of the DNA sequences, such sequences can be inserted into cells directly *in vivo,* such as by use of an appropriate viral vector. Such cells transfected *in vivo* are expected to produce antiviral amounts of cyanovirin directly *in vivo*.

Given the present disclosure, it will be additionally appreciated that a DNA sequence corresponding to a cyanovirin can be inserted into suitable nonmammalian host cells, and that such host cells will express therapeutic or prophylactic amounts of a cyanovirin directly *in vivo,* e.g., within a desired body compartment, in an animal, in particular a human.

This example further illustrates the invention but, of course, should not be construed as in any way limiting its scope.

### EXAMPLE

This example demonstrates the anti-H5N1 virus activity of cyanovirins.

Host cells used for these assays are routinely obtainable at the Southern Research Institute-Frederick (SoRI-Frederick, Frederick, Maryland). MDCK cells were used for all assays. The humanized H5N1 influenza virus strains that were used in the assays included the following: Hong Kong/491/1997; Vietnam/1203/2004H; and Hong Kong/213/2003. The bird H5N1 influenza virus strains that were used in the assays included: Duck/MN/1525/81 and Gull/PA/4175/83.

A typical antiviral assay for each virus was as follows. A pretreated aliquot of virus was removed from the freezer (-80° C) and allowed to thaw. The virus was diluted into tissue culture medium such that the amount of virus added to each well in a volume of 100 µl was that amount pre-determined to yield complete cell killing at 3-7 days (depending on the virus) post-infection. Cyanovirin-N (SEQ ID NO: 2) stock solution was diluted into the medium to the desired high concentration and then further diluted in the medium in 0.5 log10 increments.

The day following plating of cells, plates were removed from the incubator, and medium was removed and discarded. The infection medium was DMEM (Dulbecco's Minimal Essential Medium) supplemented with 0.3% BSA, 100 U/ml penicillin, 100 µg/ml streptomycin, 0.1 mM non-essential amino acids, 0.1 mM sodium pyruvate, 2 mM L-glutamine, and 2.0 µg/ml TPCK-treated (N-tosyl-L-phenylalanine ketone) trypsin. Drug dilutions (6-12 dilutions) were made in medium and added to appropriate wells of a 96-well microtiter plate in a volume of 100 µl per well. Each dilution was set up in triplicate. Infection medium containing appropriately diluted virus was added to appropriate wells of the microtiter plate. Each plate contained cell control wells (cells only), virus control wells (cells plus virus), drug toxicity control wells (cells plus drug only), drug colorimetric control wells (drug only), as well as experimental wells (drug plus cells plus virus).

After 7 days of incubation at 37° C in a 5% CO₂ incubator, the test plates were analyzed by staining with CellTiter 96^{™} AQᵤₑₒᵤₛ One Solution Reagent (Promega, Madison, WI). Twenty microliters of the reagent were added to each well of the plate, and the plate was reincubated for 4 hrs at 37° C. CellTiter 96^{™} AQᵤₑₒᵤₛ One Solution Reagent contains a tetrazolium (MTS) compound and the electron-coupling reagent phenazine ethosulfate (PES). MTS-tetrazolium is bioreduced by cells into a colored formazan product that is soluble in tissue culture medium. This conversion is mediated by NADPH and NADH produced by dehydrogenase enzymes in metabolically active cells. The amount of soluble formazan produced by cellular reduction of the MTS was determined by measuring the absorbance of light at a wavelength of 490 nm. The quantity of formazan product as determined by the amount of 490 nm wavelength light absorbance was directly proportional to the number of living cells in the culture, thus allowing the rapid quantitative analysis of the inhibition of virus-induced cell killing by the test substances.

The IC₅₀ (50% inhibition of virus replication), EC₅₀ (50% reduction in cell viability), EC₉₀ (90% reduction in cell viability), and the corresponding selectivity index (SI: IC₅₀/EC₅₀) were calculated using standard procedures. Results from testing of cyanovirin-N (SEQ ID NO: 2) against different strains of H5N1 influenza virus are shown in the table below.

**Table: Anti-H5N1 Influenza Virus Activity of Cyanovirin-N**

| **Virus Strain** | **Assay** | **EC50 (µg/mL)** | **IC50 (µg/mL)** | **EC90 (µg/mL)** | **SI** |
|---|---|---|---|---|---|
| Duck/MN/1525/81 | Virus Yield | | | 0.7 | 4 |
| | Visual | 0.5 | 2 | | 4 |
| Gull/PA/4175/83 | Virus Yield | | | 0.1 | 30 |
| | Visual | 0.1 | 3 | | 30 |
| Hong Kong/491/1997 | Virus Yield | | | 0.1 | 30 |
| | Visual | <0.0032 | 3 | | >900 |
| Hong Kong/213/2003 | Neutral Red | 0.02 | >1 | | >50 |
| | Visual | 0.01 | >1 | | >100 |
| Vietnam 1203/2004H (Trial 1) | Virus Yield | | | 0.2 | 15 |
| | Visual | 0.2 | 3 | | 15 |
| Vietnam 1203/2004H (Trial 2) | Neutral Red | 0.03 | >1 | | >33 |
| | Visual | 0.03 | >1 | | >33 |

As illustrated by the data set forth in the table, cyanovirin-N is active against all tested strains of H5N1. At the highest dose tested (1 µg/mL), cyanovirin-N was highly active against the Hong Kong/213/2003 strain and moderately active against the Hong Kong/491/1997, Vietnam, and Gull strains. Previous studies with other influenza strains indicates that cyanovirin-N causes little or no toxic side effects.

Accordingly, cyanovirin-N is effective for inhibiting an H5N1 viral infection of a host, and in particular humanized H5N1 viral infections.

The use of the terms "a" and "an" and "the" and similar referents in the context of describing the invention (especially in the context of the following claims) are to be construed to cover both the singular and the plural, unless otherwise indicated herein or clearly contradicted by context. The terms "comprising," "having," "including," and "containing" are to be construed as open-ended terms (i.e., meaning "including, but not limited to,") unless otherwise noted. Recitation of ranges of values herein are merely intended to serve as a shorthand method of referring individually to each separate value falling within the range, unless otherwise indicated herein, and each separate value is incorporated into the specification as if it were individually recited herein. All methods described herein can be performed in any suitable order unless otherwise indicated herein or otherwise clearly contradicted by context. The use of any and all examples, or exemplary language (e.g., "such as") provided herein, is intended merely to better illuminate the invention and does not pose a limitation on the scope of the invention unless otherwise claimed. No language in the specification should be construed as indicating any non-claimed element as essential to the practice of the invention.

Preferred embodiments of this invention are described herein, including the best mode known to the inventors for carrying out the invention. Variations of those preferred embodiments may become apparent to those of ordinary skill in the art upon reading the foregoing description. The inventors expect skilled artisans to employ such variations as appropriate, and the inventors intend for the invention to be practiced otherwise than as specifically described herein.

## Claims

1. Use of an antiviral protein comprising the amino acid sequence of SEQ ID NO: 1, or an antiviral portion thereof, in the preparation of a medicament for the inhibition of an H5N1 viral infection in a host.

2. Use of a nucleic acid molecule encoding an antiviral protein comprising the amino acid sequence of SEQ ID NO: 1, or an antiviral portion thereof, in the preparation of a medicament for the inhibition of an H5N1 viral infection in a host.

3. The use of claim 2, wherein the nucleic acid molecule is a vector.

4. The use of any of claims 1-3, wherein amino acid 30 of SEQ ID NO: 1 is selected from the group consisting of alanine, glutamine, and valine.

5. The use of any of claims 1-4, wherein amino acid 51 of SEQ ID NO: 1 is glycine.

6. The use of any of claims 1-5, wherein the host is a human.

7. The use of any of claim 1-5, wherein the host is a bird.

8. The use of any of claims 1-6, wherein the H5N1 virus is a humanized strain.

9. The use of any of claims 1-8, wherein the antiviral protein is part of a conjugate.

10. The use of any of claims 1-8, wherein the antiviral protein is coupled to at least one effector component.

11. The use of claim 10, wherein the effector component is selected from the group consisting of immunological reagents, toxins, and combinations thereof.

12. The use of any of claims 1-11, wherein the medicament further comprises an antiviral compound selected from the group consisting of M2 ion channel inhibitors, neuramidase inhibitors, and combinations thereof.

13. The use of claim 12, wherein the neuramidase inhibitor is selected from the group consisting of oseltamivir, zanamivir, and combinations thereof.

14. The use of claim 12 or 13, wherein the M2 ion channel inhibitor is selected from the group consisting of amandatine, rimantadine, and combinations thereof.

15. The use of any of claims 1-14, wherein the antiviral portion of the antiviral protein comprises at least nine contiguous amino acids of SEQ ID NO: 1, wherein the at least nine contiguous amino acids comprise amino acids 30-32 of SEQ ID NO: 1.

16. The use of any of claims 1-14, wherein the antiviral portion of the antiviral protein comprises at least nine contiguous amino acids of SEQ ID NO: 1, wherein the at least nine contiguous amino acids comprise amino acids 30-32 and 51 of SEQ ID NO: 1.

17. The use of claim 16, wherein amino acid 51 of SEQ ID NO: 1 is glycine.

18. The use of claim 16, wherein amino acid 51 of SEQ ID NO: 1 is deleted.

19. The use of any of claims 1-18, wherein amino acid 30 of SEQ ID NO: 1 is an amino acid selected from the group consisting of alanine, glutamine, and valine.

20. The use of any of claims 1-18, wherein amino acid 30 of SEQ ID NO: 1 is deleted.

## Patentansprüche

1. Verwendung eines antiviralen Proteins, umfassend die Aminosäuresequenz der SEQ ID NO: 1, oder eines antiviralen Anteils davon, bei der Herstellung eines Medikaments zur Hemmung einer H5N1 Virusinfektion in einem Wirt.

2. Verwendung eines Nucleinsäuremoleküls, welches ein antivirales Protein, umfassend die Aminosäuresequenz der SEQ ID NO: 1, oder einen antiviralen Anteil davon kodiert, bei der Herstellung eines Medikaments zur Hemmung einer H5N1 Virusinfektion in einem Wirt.

3. Verwendung nach Anspruch 2, wobei es sich bei dem Nucleinsäuremolekül um einen Vektor handelt.

4. Verwendung nach einem der Ansprüche 1 - 3, wobei die Aminosäure 30 der SEQ ID NO: 1 aus der Gruppe, bestehend aus Alanin, Glutamin und Valin, ausgewählt ist.

5. Verwendung nach einem der Ansprüche 1 - 4, wobei es sich bei der Aminosäure 51 der SEQ ID NO: 1 um Glycin handelt.

6. Verwendung nach einem der Ansprüche 1 - 5, wobei es sich bei dem Wirt um einen Menschen handelt.

7. Verwendung nach einem der Ansprüche 1 - 5, wobei es sich bei dem Wirt um einen Vogel handelt.

8. Verwendung nach einem der Ansprüche 1 - 6, wobei es sich bei dem H5N1 Virus um einen humanisierten Stamm handelt.

9. Verwendung nach einem der Ansprüche 1 - 8, wobei das antivirale Protein Teil eines Konjugats ist.

10. Verwendung nach einem der Ansprüche 1 - 8, wobei das antivirale Protein an mindestens eine Effektor-Komponente gekoppelt ist.

11. Verwendung nach Anspruch 10, wobei die Effektor-Komponente aus der Gruppe, bestehend aus immunologischen Reagenzien, Toxinen und Kombinationen davon, ausgewählt ist.

12. Verwendung nach einem der Ansprüche 1 - 11, wobei das Medikament ferner eine antivirale Verbindung, ausgewählt aus der Gruppe, bestehend aus M2-lonenkanalhemmern, Neuramidasehemmern und Kombinationen davon, umfasst.

13. Verwendung nach Anspruch 12, wobei der Neuramidasehemmer aus der Gruppe, bestehend aus Oseltamivir, Zanamivir und Kombinationen davon, ausgewählt ist.

14. Verwendung nach Anspruch 12 oder 13, wobei der M2-Ionenkanalhemmer aus der Gruppe, bestehend aus Amandatin, Rimantadin und Kombinationen davon, ausgewählt ist.

15. Verwendung nach einem der Ansprüche 1 - 14, wobei der antivirale Anteil des antiviralen Proteins mindestens neun zusammenhängende Aminosäuren der SEQ ID NO: 1 umfasst, wobei die mindestens neun zusammenhängenden Aminosäuren die Aminosäuren 30 - 32 der SEQ ID NO: 1 umfassen.

16. Verwendung nach einem der Ansprüche 1 - 14, wobei der antivirale Anteil des antiviralen Proteins mindestens neun zusammenhängende Aminosäuren der SEQ ID NO: 1 umfasst, wobei die mindestens neun zusammenhängenden Aminosäuren die Aminosäuren 30 - 32 und 51 der SEQ ID NO: 1 umfassen.

17. Verwendung nach Anspruch 16, wobei es sich bei der Aminosäure 51 der SEQ ID NO: 1 um Glycin handelt.

18. Verwendung nach Anspruch 16, wobei die Aminosäure 51 der SEQ ID NO: 1 deletiert ist.

19. Verwendung nach einem der Ansprüche 1 - 18, wobei es sich bei der Aminosäure 30 der SEQ ID NO: 1 um eine Aminosäure, ausgewählt aus der Gruppe, bestehend aus Alanin, Glutamin und Valin, handelt.

20. Verwendung nach einem der Ansprüche 1 - 18, wobei die Aminosäure 30 der SEQ ID NO: 1 deletiert ist.

## Revendications

1. Utilisation d'une protéine antivirale comprenant la séquence d'acides aminés de SEQ ID N° 1, ou une portion antivirale de celle-ci, pour la préparation d'un médicament pour l'inhibition d'une infection virale de H5N1 chez un hôte.

2. Utilisation d'une molécule d'acide nucléique codant pour une protéine antivirale comprenant la séquence d'acides aminés de SEQ ID N° 1, ou une portion antivirale de celle-ci, pour la préparation d'un médicament pour l'inhibition d'une infection virale de H5N1 chez un hôte.

3. Utilisation selon la revendication 2, dans laquelle la molécule d'acide nucléique est un vecteur.

4. Utilisation selon l'une quelconque des revendications 1 à 3, dans laquelle l'acide aminé 30 de SEQ ID N° 1 est choisi parmi le groupe constitué d'alanine, de glutamine et de valine.

5. Utilisation selon l'une quelconque des revendications 1 à 4, dans laquelle l'acide aminé 51 de SEQ ID N° 1 est la glycine.

6. Utilisation selon l'une quelconque des revendications 1 à 5, dans laquelle l'hôte est un humain.

7. Utilisation selon l'une quelconque des revendications 1 à 5, dans laquelle l'hôte est un oiseau.

8. Utilisation selon l'une quelconque des revendications 1 à 6, dans laquelle le virus H5N1 est une souche humanisée.

9. Utilisation selon l'une quelconque des revendications 1 à 8, dans laquelle la protéine antivirale est une partie d'un conjugué.

10. Utilisation selon l'une quelconque des revendications 1 à 8, dans laquelle la protéine antivirale est couplée à au moins un composant effecteur.

11. Utilisation selon la revendication 10, dans laquelle le composant effecteur est choisi parmi le groupe constitué de réactifs immunologiques, de toxines et de combinaisons de ceux-ci.

12. Utilisation selon l'une quelconque des revendications 1 à 11, dans laquelle le médicament comprend en outre un composé antiviral choisi parmi le groupe constitué d'inhibiteurs de canaux ioniques M2, d'inhibiteurs de neuraminidase et de combinaisons de ceux-ci.

13. Utilisation selon la revendication 12, dans laquelle l'inhibiteur de la neuraminidase est choisi parmi le groupe constitué d'oseltamivir, de zanamivir et de combinaisons de ceux-ci.

14. Utilisation selon la revendication 12 ou 13, dans laquelle l'inhibiteur du canal ionique M2 est choisi parmi le groupe constitué d'amandatine, de rimantadine et de combinaisons de celles-ci.

15. Utilisation selon l'une quelconque des revendications 1 à 14, dans laquelle la portion antivirale de la protéine antivirale comprend au moins neuf acides aminés contigus de SEQ ID N° 1, dans laquelle au moins neuf acides aminés contigus comprennent des acides aminés 30 à 32 de SEQ ID N° 1.

16. Utilisation selon l'une quelconque des revendications 1 à 14, dans laquelle la portion antivirale de la protéine antivirale comprend au moins neuf acides aminés contigus de SEQ ID N° 1, dans laquelle au moins neuf acides aminés contigus comprennent des acides aminés 30 à 32 et 51 de SEQ ID N° 1.

17. Utilisation selon la revendication 16, dans laquelle l'acide aminée 51 de SEQ ID N° 1 est la glycine.

18. Utilisation selon la revendication 16, dans laquelle l'acide aminé 51 de SEQ ID N°1 est supprimé.

19. Utilisation selon l'une quelconque des revendications 1 à 18, dans laquelle l'acide aminé 30 de SEQ ID N° 1 est un acide aminé choisi parmi le groupe constitué d'alanine, de glutamine et de valine.

20. Utilisation selon l'une quelconque des revendications 1 à 18, dans laquelle l'acide aminé 30 de SEQ ID N°1 est supprimé.
